# EUROPEAN PATENT APPLICATION

(11) **EP 3 162 890 A1**
(43) Date of publication of application: **03.05.2017**
(21) Application number: 16195338.5
(22) Date of filing: 24.10.2016
(51) Int. Cl.: C12N 5/00

(54) **METHOD AND APPARATUS FOR PRODUCING CELL MASS STRUCTURE**

(30) Priority: 29.10.2015 JP 2015212911; 09.09.2016 JP 2016176819
(71) Applicant: Shibuya Corporation, Kanazawa-shi, Ishikawa 920-8681 (JP)
(72) Inventor: YONEDA, Kenji, Kanazawa-shi, Ishikawa 920-8681 (JP); KOSHIDA, Ichiro, Kanazawa-shi, Ishikawa 920-8681 (JP); HATANAKA, Akira, Kanazawa-shi, Ishikawa 920-8681 (JP); NAGAI, Hiroyuki, Kanazawa-shi, Ishikawa 920-8681 (JP)
(74) Representative: Hodsdon, Stephen James

(57) **Abstract**

The present invention relates to a method for producing a cell mass structure, which produces a cell mass structure (adhesion pad 4) in which a plurality of cell aggregates 2 arranged inside a culture container 3 accommodating a culture liquid are fused to each other by being cultured. A plurality of pins 16 are erected inside the above-described culture container 3, the above-described cell aggregates 2 are accommodated by the plurality of pins 16 with parts between the plurality of pins 16 and pins 16 as accommodating portions H, and the cell aggregates 2 are cultured in a state of being accommodated in the plurality of adjacent accommodating portions H. It is possible to obtain a required shape and dimension, and the cell mass structure can be obtained without giving damage to cells further.

## Description

### BACKGROUND OF THE INVENTION

### Field of the Invention

The present invention relates to a method and an apparatus for producing a cell mass structure, and specifically relates to the method and the apparatus for producing the cell mass structure in which a plurality of cell aggregates arranged inside a culture container accommodating a culture liquid are cultured and fused to each other.

### Description of the Related Art

In regenerative medical techniques of renaturing vital functions that have received damage, by using a stem cell or the like, a cell sheet is used that is formed of cells which are cultured into high density. The cell sheet is extremely thin, and accordingly a method for producing a layered sheet of cultured cells is known in which several cell sheets are layered (Japanese Patent No. 4921353).

In addition, on the other hand, a method for producing sheet-like cell aggregates having a thickness of 50 to 300 µm is known, in which cell aggregates (spheroids) that have been formed each into an approximately spherical shape by being cultured are arrayed in a state of coming in contact with each other are fused to each other by being subjected to float culture (Japanese Patent No. 5523830).

Furthermore, a method for producing a three-dimensional structure of cells is known, which makes a support formed of a thread-like body or a needle-like body penetrate cell aggregates (Japanese Patent No. 4517125), as a method of arranging cell aggregates in an arbitrary space.

However, in the case of layering the cell sheets as in Japanese Patent No. 4921353, the cell sheet is difficult to handle and the automation has been difficult, because of being extremely thin.

In addition, in the case of intending to obtain the sheet-like cell aggregates by subjecting the cell aggregates to float culture as in Japanese Patent No. 5523830, arrayed cell aggregates become sparse dense, the cell aggregates are irregularly fused to each other, and accordingly there have been problems that the shape of the obtained sheet-like cell aggregates is not constant, and also the thickness becomes nonuniform.

Furthermore, in the case of making the support formed of the thread-like body or the needle-like body penetrate the cell aggregates as in Japanese Patent No. 4517125, there has been a possibility of giving damage to the cell.

With respect to such problems, the present invention provides a method and an apparatus for producing a cell mass structure, which can obtain a required shape and dimension and do not give damage to the cell.

### SUMMARY OF THE INVENTION

That is, a method for producing a cell mass structure according to the invention of claim 1 is the method for producing a cell mass structure which produces a cell mass structure in which a plurality of cell aggregates arranged inside a culture container accommodating a culture liquid are fused to each other by being cultured, and is characterized in that
a plurality of pins are erected inside the culture container, and the cell aggregates are accommodated by the plurality of pins with parts between the plurality of pins and pins as accommodating portions, and
the cell aggregates are cultured in a state of being accommodated in the plurality of adjacent accommodating portions.

In addition, an apparatus for producing a cell mass structure according to the invention of claim 7 is the apparatus for producing a cell mass structure, which produces a cell mass structure in which a plurality of cell aggregates arranged inside a culture container accommodating a culture liquid are fused to each other by being cultured, and is characterized by comprising:
accommodating means with a plurality of pins erected and provided inside the culture container; and
supply means that supplies the cell aggregates between the plurality of pins and pins in the accommodating means further, and
with parts between the plurality of pins and pins as the accommodating portions of the cell aggregates, the supply means accommodates the aggregates in the plurality of adjacent accommodating portions.

According to the invention of claim 1 and claim 7, since, with the parts between the plurality of pins and pins as accommodating portions, the cell aggregates are accommodated in the plurality of adjacent accommodating portions, the individual cell aggregates are fused to each other by being cultured in the accommodating portions, and the cell mass structure of the required shape and dimension can be produced. In addition, since it is not needed to make the pin penetrate the cell aggregate, damage to cells can be suppressed.

### BRIEF DESCRIPTION OF THE DRAWINGS

FIG. 1 is a front view of an isolator and an incubator in which an apparatus for producing a cell mass structure is provided;
FIG. 2 is diagrams for describing structure of the apparatus for producing a cell mass structure;
FIG. 3 is a cross-sectional view of an accommodating container and a suction nozzle;
FIG. 4 is cross-sectional views of a culture container;
FIG. 5 is a plan view of cell aggregates accommodated in the culture container;
FIG. 6 is a side view of culture liquid supply means;
FIG. 7 is diagrams for describing handling of a cell mass structure inside the culture container in a second example;
FIG. 8 is a diagram for describing handling of a cell mass structure inside the culture container in a third example;
FIG. 9 is a diagram for describing arrangement of pins structuring first and second pin groups in a fourth example;
FIG. 10 is diagrams for describing handling of a cell mass structure inside the culture container in the fourth example;
FIG. 11 is a diagram for describing another structure of the first and second pin groups in the fourth example;
FIG. 12 is diagrams for describing another structure of the first and second pin groups in the fourth example;
FIG. 13 is diagrams for describing handling of a cell mass structure inside the culture container in a fifth example;
FIG. 14 is a diagram for describing a step that can be added to the fifth example;
FIG. 15 is a diagram for describing handling of a cell mass structure inside the culture container in a sixth example;
FIG. 16 is a diagram for describing arrangement of cell aggregates inside the culture container in the sixth example; and
FIG. 17 is diagrams for describing another method of holding cell aggregates inside the culture container.

### DETAILED DESCRIPTION OF THE PREFERRED EMBODIMENTS

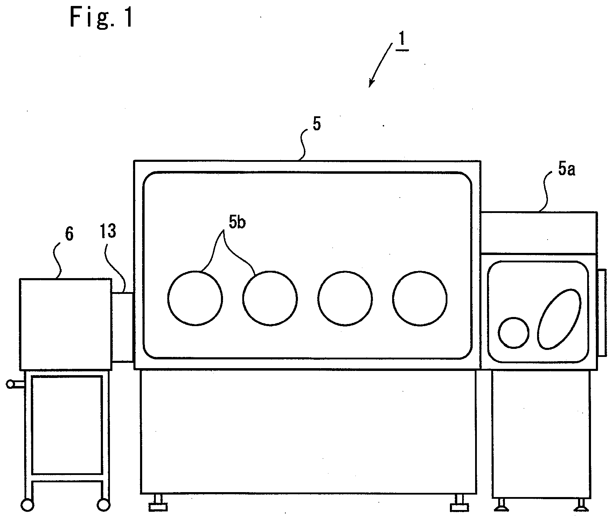

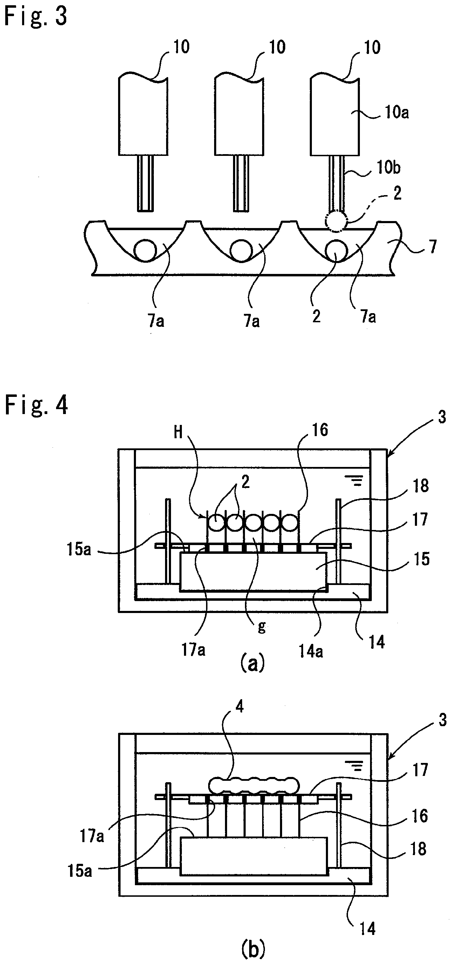

Illustrated examples will be described below. FIG. 1 to FIG. 6 are views for describing an apparatus 1 for producing a cell mass structure according to a first example, which is structured so as to produce an adhesion pad 4 (see FIG. 4) as a cell mass structure in which a plurality of cell aggregates 2 (spheroids: see FIG. 3) are arranged inside a culture container 3, and the above-described cell aggregates 2 are fused to each other by being cultured.

The above-described cell aggregates 2 can be produced, for instance, by a method which is disclosed in Japanese Patent No. 4517125. Specifically, when the cells are seeded and cultured in a container of which the inner surface is non-adhesive, the cells seek scaffolds, adhere to each other, and thereby form the cell aggregates 2. The cell aggregates are further fused and thereby the cell aggregates 2 are formed that have an approximately spherical shape of which the outer diameter has a dimension of approximately 500 µm.

More efficiently, the cell aggregates 2 are cultured in a non-adhesive well (accommodating portion having approximately hemispherical shape) in a well plate, and thereby can be more easily obtained. Incidentally, the method for producing the cell aggregate 2 is not limited to the above method, and can be produced by various known methods such as a rotary culture method of charging a cell suspension liquid into a rotating culture liquid, a method of charging a cell suspension liquid in a test tube and making the cells settle by a centrifugal separator, and a method of culturing cells with the use of alginate beads.

Thus, the cell aggregate 2 means a cell assembly that is formed of cells which have aggregated and agglomerated to each other, and has an approximately spherical shape of which the outer diameter has a dimension of approximately 100 to 500 µm. When the cell aggregates 2 are planarly or three-dimensionally brought into contact or made to approach and arranged, the cells of the individual cell aggregates 2 increase and are fused with the adjacent cell aggregates 2, and a planar or three-dimensional cell mass structure is obtained.

The adhesion pad 4 produced in the present example can be used to adhere to a cartilage or the like, and the plurality of cell aggregates 2 are planarly arranged and fused. As for a thickness, the pad can be produced which has the thickness of approximately 100 to 500 µm depending on the dimension of the cell aggregate 2 to be used, and the planar size can be adjusted by the number of the cell aggregates 2 to be arrayed.

The above-described adhesion pad 4 that is produced in this way is different from a cell sheet that is obtained by culturing single cells into a sheet shape by planarly culturing a cell suspension liquid.

The apparatus 1 for producing a cell mass structure in the present example is provided in the inner parts of an isolator 5 of which the inner part is kept in a sterile condition, and of an incubator 6 which is provided so as to be capable of being connected to the isolator 5, which are shown in FIG. 1. In addition, a pass box 5a for decontaminating an object to be carried in is provided in the isolator 5.

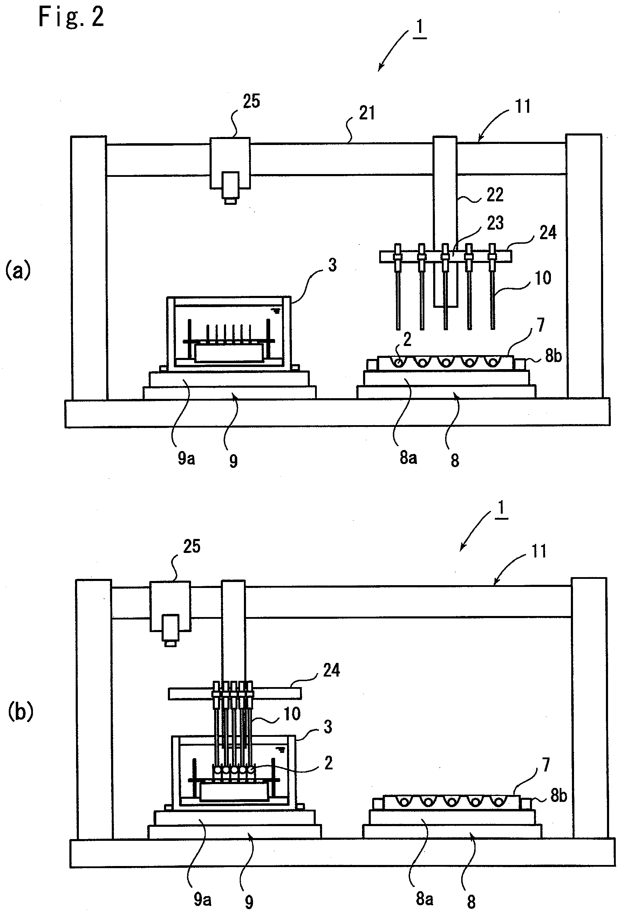

FIG. 2 shows a structure provided in the inner part of the above-described isolator 5. The structure includes: an accommodating container supporting section 8 that supports an accommodating container 7 which accommodates the cell aggregate 2 therein; a culture container supporting section 9 that supports the culture container 3 in which the adhesion pad 4 is cultured; a plurality of suction nozzles 10 which hold the above-described cell aggregate 2; and nozzle moving means 11 which moves the above-described suction nozzle 10 relatively to the above-described accommodating container 7 and culture container 3.

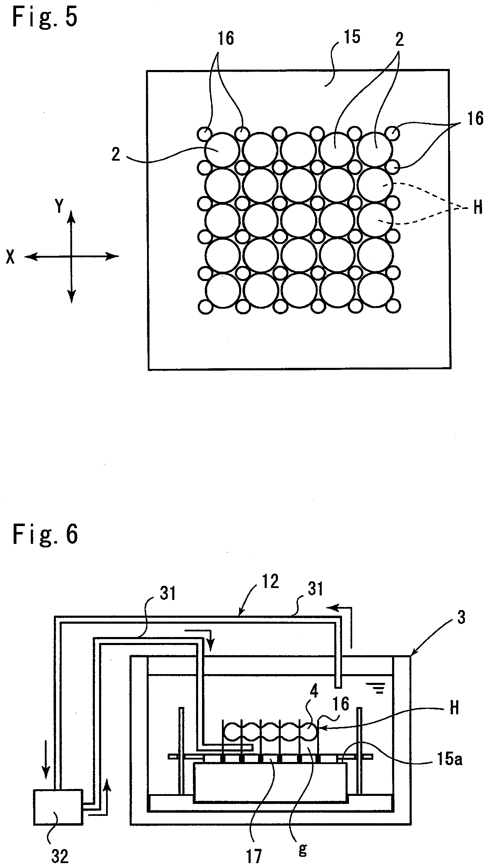

On the other hand, FIG. 6 shows a structure provided in the inner part of the above-described incubator 6. The structure includes culture liquid supply means 12 which supplies the culture liquid to the cell aggregates 2 or the adhesion pad 4 on the culture container 3.

Incidentally, in the following description, a left-right direction is defined as an X direction, a front-rear direction is defined as a Y direction, and an upper-lower direction is defined as a Z direction, in FIG. 2; and in FIG. 5, a left-right direction is defined as the X direction, and an upper-lower direction is defined as the Y direction.

In the above-described isolator 5, the inside is kept in a sterile environment, and a flow of sterile air in one direction, which heads to the lower part from the upper part, is formed by sterile air supply means.

In addition, gloves 5b that an operator can wear are provided on the front face of the isolator 5 so that the operator can perform various works. Incidentally, it is also possible to provide a robot or transfer means having a required structure in the inside of the isolator 5, and make these works automatically performed.

Next, in the above-described incubator 6, the inside may be kept in a sterile environment, and also may be kept at a predetermined temperature and humidity that are suitable for culture of the adhesion pad 4; and the above-described incubator 6 may be structured to be separated from the above-described isolator 5 and mounted on a place separated from the isolator 5 while the cell aggregates 2 are fused to each other and are formed into the adhesion pad 4.

Because of this, the above-described isolator 5 and incubator 6 are connected to each other by connection means 13, and connection means can be used that contacts/separates the incubator 6 with/from the isolator 5 while the sterile condition is kept, as is described, for instance, in Japanese Patent No. 4656485.

FIG. 3 shows a cross-sectional view of the above-described accommodating container 7, and the above-described well plate can be used. The accommodating container 7 has a plurality of recessed accommodating portions 7a provided lengthwise and breadthwise when being viewed as the plane, and the cell aggregates 2 each having an approximately spherical shape are accommodated in the inside of the recessed accommodating portions 7a together with the culture liquid, one by one respectively.

A predetermined number of the recessed accommodating portions 7a in the above-described accommodating container 7 are arrayed each in the X-direction and the Y-direction when being viewed as the plane, and in the present example, the same number as the number of the cell aggregates 2 that constitute the adhesion pad 4 which is produced in the culture container 3, in the X-direction and the Y-direction, specifically, five recessed accommodating portions 7a in the X direction and five recessed accommodating portions 7a in the Y direction are provided, respectively.

The accommodating container supporting section 8 which supports the above-described accommodating container 7 positions the above-described accommodating container 7 on the upper face thereof by a positioning piece 8b, and also is formed of a Y-direction table 8a which constitutes the above-described nozzle moving means 11.

As will be described later, in the present example, the five suction nozzles 10 are provided in the X direction and are structured so as to be capable of moving only in the X direction and the Z direction; and accordingly, the suction nozzles 10 and the accommodating container 7 can be relatively moved in each of the directions of X, Y and Z, by causing the above-described Y-direction table 8a to move the accommodating container 7 in the Y-direction.

The above description will be specifically described below. The above-described Y-direction table 8a is structured so as to firstly position the recessed accommodating portions 7a of the first row of the accommodating container 7 below the suction nozzles 10, and when the suction nozzles 10 adsorb the cell aggregates 2 from the recessed accommodating portions 7a of the first row and hold those thereon, move the accommodating container 7 in the Y-direction by one row to position the recessed accommodating portions 7a of the second row below the suction nozzles 10.

As is shown in FIG. 4, the culture container 3 has a closed-end box shape, accommodates the culture liquid therein up to a predetermined depth, and has a bottom face plate 14 installed to a bottom face, accommodating means formed of a pedestal 15 installed to the bottom face plate 14 and a plurality of pins 16 erected on the pedestal 15, and a support plate 17 as a lower support member which is moved up and down along the pins 16.

The above-described bottom face plate 14 is in an approximately same shape as that of the bottom face of the culture container 3, and the bottom face plate 14 is positioned so as not to move inside the culture container 3. In addition, the pedestal 15 has a rectangular parallelepiped shape, and is fitted and positioned to a recessed portion 14a formed on the upper face of the bottom face plate 14.

The above-described pins 16 are regularly erected on an installation surface 15a formed on the upper face of the pedestal 15 so as to turn to the Z direction respectively.

FIG. 5 shows a plan view of the pins 16 provided on the pedestal 15 and the cell aggregates 2 held by the pins 16. In the present example, five cell aggregates 2 each in the X direction and the Y direction are planarly arrayed in the state of being in contact with each other.

The above-described pins 16 are provided so as to be positioned around the individual cell aggregates 2, and in the present example, an accommodating portion H which accommodates the cell aggregate 2 is structured by four pins 16. More specifically, an approximately square section, at four corners of which the four pins 16 are positioned, is the above-described accommodating portion H when being viewed as the plane.

In the present example, arrangement is performed such that, of the four pins 16 structuring the above-described accommodating portion H, an interval of the two pins 16 at individual diagonal positions becomes shorter than a diameter of the cell aggregate 2 to be accommodated. In other words, when producing the cell aggregate 2, the diameter is made larger than the interval of the two pins 16 at the individual diagonal positions.

When the cell aggregate 2 is accommodated in the accommodating portion H structured in this way, the cell aggregate 2 can be held between the plurality of pins 16 and pins 16, and it becomes possible to position the cell aggregate 2 at a vertical middle part between a distal end and a root of the above-described pins 16 as is shown in FIG. 4(A).

In other words, the above-described accommodating portion H can be set at a position separated from the installation surface 15a where the plurality of pins 16 are erected, and a gap g through which the culture liquid can be distributed can be formed between the cell aggregate 2 held by the accommodating portion H and the installation surface 15a.

In addition, though it depends on the diameter of the pin 16, by making the interval of the two pins 16 at the individual diagonal positions shorter than the diameter of the cell aggregate 2, the cell aggregates 2 in the individual accommodating portions H can be cultured in the state of being brought into contact with each other.

The above-described bottom face plate 14 is provided with two rod-like guide members 18 which are provided on positions facing each other across the pedestal 15, are erected in the Z direction and vertically penetrate the above-described support plate 17, and through-holes 17a through which the above-described pins 16 penetrate are bored in the support plate 17.

By the above-described structure, the support plate 17 is provided so as to be raised and lowered in the Z direction along the above-described pins 16 and the guide members 18, and is normally positioned at a lowered position to be mounted on the installation surface 15a of the above-described pedestal 15 by gravity.

When the support plate 17 is positioned at the lowered position as is shown in FIG. 4(A), the gap g through which the culture liquid can be distributed is formed between the cell aggregates 2 held at the vertical middle part of the above-described pins 16 and the installation surface 15a of the above-described pedestal 15.

On the other hand, as is shown in FIG. 4(B), when the support plate 17 is raised, the support plate 17 can support the cell aggregates 2 held between the above-described pins 16 and pins 16 from below, and since the support plate 17 is positioned at a raised position, the adhesion pad 4 in which the cell aggregates 2 are cultured can be taken out from between the above-described pins 16.

The above-described support plate 17 can be raised by an operator wearing the gloves 5b and can also be raised by a robot or the like. At the time, though not shown in the figure, by providing a grasping member projected upwards from a liquid level of the above-described culture liquid on an upper face of the above-described support plate 17, it is made possible to raise the support plate 17 without touching the culture liquid.

As is shown in FIG. 2, the culture container supporting section 9 includes a Y-direction table 9a which constitutes the above-described nozzle moving means 11, and is structured so as to move the whole culture container 3 that contains the accommodating portions H set between the pins 16, in the above-described Y-direction one row by one row, every time the cell aggregates 2 that have been adsorbed and held by the suction nozzles 10 are supplied to the accommodating portions H constituted by the above-described pins 16, similarly to the Y-direction table 8a that constitutes the above-described accommodating container supporting section 8.

Next, the suction nozzle 10 will be described. As is shown in FIG. 3, the suction nozzle 10 has a main body portion 10a that is connected to not-shown negative pressure generating means, and an adsorbing portion 10b that has a tubular shape and is provided beneath the bottom end of the main body portion 10a.

The inner diameter of the above-described adsorbing portion 10b becomes a smaller diameter than that of the above-described cell aggregate 2, and is formed so that the cell aggregate 2 is not sucked into the inside of the adsorbing portion 10b, when the tip of the adsorbing portion 10b has adsorbed and held the cell aggregate 2 thereon, in the recessed accommodating portion 7a of the above-described accommodating container 7.

In addition, in the culture container 3, in the state that a central position of the above-described adsorbing portion 10b is positioned in the approximately center of the accommodating portion H that is formed between the pin 16 and the pin 16 and the cell aggregate 2 is positioned at the vertical middle position of the above-described pins 16, a negative pressure by the above-described negative pressure generating means is canceled.

Incidentally, instead of the suction nozzle 10 which adsorbs and holds the cell aggregate 2 thereon, means which has such a structure as to hold the cell aggregate 2 with a gripper or the like may be used to hold the cell aggregate 2.

In addition, the nozzle moving means 11 which moves the above-described suction nozzles 10 includes: an X-direction rail 21 that is provided in the X direction from the above-described accommodating container supporting section 8 to the above-described culture container supporting section 9; X-direction moving means 22 that moves the suction nozzles 10 in the X direction along the X-direction rail 21; Z-direction moving means 23 that is provided on the X-direction moving means 22 and moves the suction nozzles 10 up and down in the Z direction; and the above-described Y-direction tables 8a and 9a on the above-described accommodating container supporting section 8 and the above-described culture container supporting section 9. Then, the suction nozzles 10 and the nozzle moving means 11 constitute supply means of the cell aggregates 2.

Incidentally, instead of the Y-direction tables 8a and 9a, the nozzle moving means 11 may be the one which has a structure of moving the above-described X-direction rail 21 in the Y direction. By such a structure, the above-described suction nozzles 10 and a camera 25 described later can be moved in the X-Y direction.

In addition, the five suction nozzles 10 in the present example are provided in the X direction in the state of being arrayed, and are structured so that the spaces can be changed in the X direction by space changing means 24.

Specifically, when the above-described suction nozzles 10 are positioned above the accommodating container supporting section 8, the space changing means 24 changes the spaces between the above-described suction nozzles 10 to the same spaces as those between the recessed accommodating portions 7a that are arrayed in the X direction in the above-described accommodating container 7.

On the other hand, when the suction nozzles 10 are positioned above the culture container supporting section 9, the space changing means 24 changes the spaces between the suction nozzles 10 to the same spaces as the spaces in the X direction between the accommodating portions H that are formed between the pin 16 and the pin 16 inside the above-described culture container 3.

Incidentally, it is also possible to provide a plurality of rows of the suction nozzles 10 in the Y direction, and when the spaces between the recessed accommodating portions 7a on the accommodating container supporting section 8 are the same as the spaces between the accommodating portions H that are formed between the pin 16 and the pin 16, the above-described space changing means 24 can be omitted.

In addition, for instance, when the spaces between the above-described accommodating portions H are narrow, it is also possible to accommodate the cell aggregates 2 in every other accommodating portions H by the above-described suction nozzles 10, and in this case, firstly, it is acceptable to mount the cell aggregates 2 in the odd-numbered accommodating portions H, and then to mount the cell aggregates 2 in the even-numbered accommodating portions H.

Incidentally, at least one row of suction nozzles 10 may be provided, and the supply means of the cell aggregates 2 may have, in addition to a structure in which the suction nozzles move and mount the cell aggregates 2 as in the present example, such a structure as to send out the cell aggregates 2 one by one from the container in which a large number of cell aggregates 2 are accommodated.

In addition, on the above-described X-direction rail 21, the camera 25 is provided that moves in the X direction, and the above-described camera 25 is structured so as to move to the upper part of the culture container supporting section 9, and to photograph the accommodating portions H inside the culture container 3, when the cell aggregates 2 are held in the accommodating portions H by the above-described suction nozzles 10.

It is checked whether or not the cell aggregates 2 are held in all of the accommodating portions H by a photographed image, and it is possible to give a direction to supply the cell aggregates 2 to the accommodating portions H which do not hold the cell aggregates 2 as needed.

In addition, the camera 25 is structured, when the above-described nozzle moving means 11 makes the suction nozzles 10 positioned above the culture container supporting section 9, so as to retract from the upper part of the culture container supporting section 9, in order to avoid the contact with the suction nozzle 10.

FIG. 6 shows the culture liquid supply means 12 that supplies the culture liquid to the adhesion pad 4 in the middle of culture, which is provided in the inside of the incubator 6, and when the plurality of culture containers 3 are accommodated in the inside of the above-described incubator 6, the above-described culture liquid supply means 12 can be provided on each of the culture containers 3.

The above-described culture liquid supply means 12 includes a pipe 31 of which both ends are inserted into the liquid level of the culture liquid, from above the above-described culture container 3, and a liquid sending pump 32 that is provided in the pipe 31 and sends the culture liquid.

One end of the pipe 31 is positioned near the liquid level of the culture liquid in the above-described culture container 3, that is, above the cell aggregates 2 held between the above-described pins 16, and the other end is positioned in the gap g formed between the above-described cell aggregates 2 and the installation surface 15a of the above-described pedestal 15 in the above-described culture container 3.

Then, the above-described liquid sending pump 32 is structured so as to suck the culture liquid from the upper side in the culture container 3 from one end of the pipe 31, also make the culture liquid flow into the above-described gap g from the other end, and thereby make the culture liquid circulate in the culture container 3.

By such a structure, the culture liquid supplied by the above-described culture liquid supply means 12 is supplied to the bottom face side of the adhesion pad 4 with the above-described gap g formed below, the culture liquid sufficiently spreads even to the bottom face side of the adhesion pad 4, and thereby the culture can be adequately performed.

Incidentally, a liquid-sending direction of the liquid sending pump 32 may be reversed such that the culture liquid is sucked from the other end of the above-described pipe 31 positioned in the above-described gap g, and in that case, since a flow through which the culture liquid flows from the periphery of the adhesion pad 4 into the bottom face side is formed, the culture liquid is supplied to the above-described gap g.

In addition, as preprocessing of the culture liquid supply operation, nutrients of the culture liquid may be replenished, oxygen and carbon dioxide may be supplied and pH may be adjusted as needed.

A method for producing the adhesion pad 4 with the use of the apparatus 1 for producing the cell mass structure, which has the above-described structure, will be described below.

Firstly, a preparation step is performed that carries the accommodating container 7 in which the above-described cell aggregates 2 are accommodated and the culture container 3 into the inside of the above-described isolator 5 through the above-described pass box 5a, and supplies a predetermined amount of the culture liquid to the culture container 3.

In addition, the accommodating means formed of the above-described pedestal 15 and the plurality of pins 16 is installed in the culture container 3, and the above-described support plate 17 is positioned at the lowered position in contact with the installation surface 15a of the above-described pedestal 15 by gravity.

Further, in the preparation step, if the plurality of above-described accommodating containers 7 and culture containers 3 are carried into the inside of the isolator 5, it becomes possible to continuously produce the plurality of adhesion pads 4 by using the plurality of culture containers 3.

When the above-described preparation step is completed, subsequently, a supply step is performed that moves the above-described suction nozzles 10, moves the cell aggregates 2 to the above-described culture container 3, and supplies the cell aggregates 2 to the accommodating portions H set between the above-described pins 16.

The above-described nozzle moving means 11 moves the suction nozzles 10, the space changing means 24 changes the spaces between the suction nozzles 10, and the cell aggregates 2 are adsorbed and held from the recessed accommodating portions 7a in the accommodating container 7 in the above-described accommodating container supporting section 8.

Subsequently, the nozzle moving means 11 moves the suction nozzles 10 to the culture container supporting section 9, and the space changing means 24 changes the spaces between the suction nozzles 10 in accordance with the spaces between the accommodating portions H formed between the above-described pins 16 and pins 16, and lowers the suction nozzles 10.

Then, each cell aggregate 2 held by the suction nozzle 10 is inserted to the accommodating portion H formed by the four pins 16, and the cell aggregate 2 is held at the vertical middle position of the above-described pins 16 such that the gap g is formed below the cell aggregate 2.

At the time, since the cell aggregate 2 accommodated in the above-described accommodating portion H is held by the four pins 16 that surround the periphery, an arrangement state of the cell aggregates 2 can be maintained without a lift of the cell aggregate 2 from the accommodating portion H as is shown in FIG. 4(A), and it is possible to maintain the state that the above-described gap g is formed between the cell aggregates 2 and the installation surface 15a of the pedestal 15.

When the cell aggregates 2 have been accommodated in all of the accommodating portions H in this way, the above-described camera 25 then moves to the upper part of the culture container supporting section 9, photographs the cell aggregates 2 inside the culture container 3, and checks whether or not the cell aggregates 2 are accommodated in all of the accommodating portions H.

Thus, when the cell aggregates 2 have been arranged inside the culture container 3, the above-described culture container 3 is transferred to the incubator 6 by the robot or manual work of the operator, and is subjected to a culture step that cultures the cell aggregates 2 inside the culture container 3.

Firstly, when the above-described culture container 3 is transferred to the incubator 6, the operator installs each of both ends of the pipe 31 of the above-described culture liquid supply means 12 inside the culture container 3, specifically, inserts one end near the liquid level of the culture liquid and the other end to the gap g below the cell aggregates 2 held between the pins 16.

Thereafter, the incubator 6 is separated from the above-described isolator 5, and is mounted on a position that is separated from the isolator 5, the inside is kept at a predetermined temperature and a predetermined humidity, and predetermined concentrations of carbon dioxide and oxygen are kept.

Inside the culture container 3, when the cell aggregates 2 are cultured, the adjacent cell aggregates 2 are fused to each other, and thus the adhesion pad 4 can be obtained as the cell mass structure which keeps the shape that the cell aggregates 2 are planarly arranged.

That is, it is possible to product the adhesion pad 4 in an arbitrary shape just as the cell aggregates 2 are arranged, and since the cell aggregates 2 are arranged without a gap by the above-described accommodating portions H, the thickness of the adhesion pad 4 which is planarly formed by the fusion of the above-described cell aggregates 2 becomes approximately uniform.

In addition, since the movement of each cell aggregate 2 is regulated by the above-described pins 16, the dimension of the adhesion pad 4 to be formed can be approximately the same as a size when the cell aggregates 2 are arranged first.

This is because that the cell aggregates 2 increase or move so as to be closely attached to the periphery of the above-described pins 16 in contact, and fill clearances formed between the adjacent cell aggregates 2, and the overall dimension is hardly contracted as in the case that the cell aggregates 2 are planarly arranged and cultured without interposing the pins 16.

During the above-described culture step, a culture liquid supply step is performed that supplies the culture liquid to the cell aggregates 2 or the adhesion pad 4 held in the accommodating portions H at a predetermined interval inside the incubator 6.

As the above-described culture liquid supply step, the culture liquid supply means 12 makes the culture liquid circulate inside the culture container 3, and since the above-described gap g is formed below the above-described cell aggregates 2 at that time, the culture liquid is supplied between the adhesion pad 4 and the installation surface 15a of the pedestal 15, and the back face side of the adhesion pad 4 can be efficiently cultured.

That is, when the cell aggregates 2 are mounted on the above-described installation surface 15a and the adhesion pad 4 is produced, there has been a problem in that the culture liquid does not sufficiently spread to the back face side of the cell aggregates 2 which are positioned in the middle, but by holding the cell aggregates 2 in the state that the gap g is formed below, it has become possible to adequately culture also the cells which are positioned in the portion.

When the cell aggregates 2 are cultured by the above-described culture step and the adhesion pad 4 is prepared, the incubator 6 is connected to the isolator 5 and a collection step is performed that collects the adhesion pad 4 from the culture container 3.

In the collection step, the above-described culture container 3 is supported by the culture container supporting section 9, and in the state, the above-described support plate 17 is raised in the Z direction and positioned at the raised position inside the above-described culture container 3.

The support plate 17 comes in contact with the lower part of the adhesion pad 4 while being raised, supports the adhesion pad 4 from below as it is, raises the adhesion pad 4 along the pins 16 and the guide members 18, and thus detaches the adhesion pad 4 from between the pins 16.

As is shown in FIG. 4(B), when the support plate 17 is positioned at the raised position and the adhesion pad 4 is detached from between the pins 16, the adhesion pad 4 on the support plate 17 can be transferred to a required container and collected.

At the time, since the adhesion pad 4 is not closely attached to the support plate 17 in the culture step, the adhesion pad 4 is not stuck to the support plate 17, and can be easily collected.

Incidentally, from the above-described culture step to the collection step, the incubator 6 may be connected to the isolator 5 at every predetermined period to perform an inspection step that inspects the cultured state of the adhesion pad 4 in the middle of the culture.

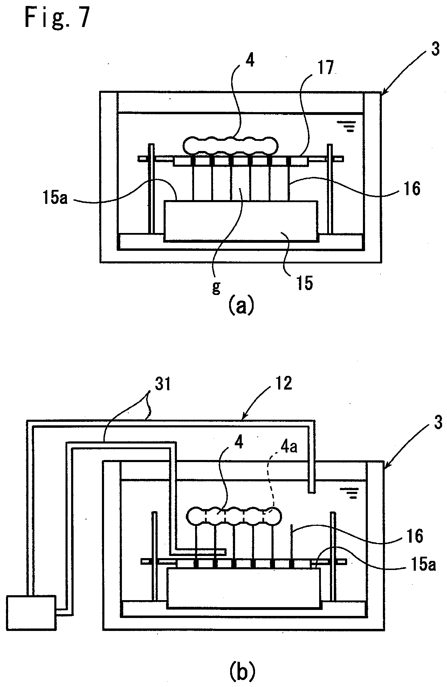

FIG. 7 is diagrams for describing a method for producing a cell mass structure as a second example, and is specifically work that is executed in the middle of the above-described culture step in the above-described first example, and the same elements are used for the culture container 3 and the like.

The work relating to the present example is performed after the above-described culture step is started in the incubator 6, then the cell aggregates 2 are turned to the state of being fused to each other, and the adhesion pad 4 in the middle of the culture is formed.

The adhesion pad 4 in the middle of the culture is in the state that the above-described pins 16 penetrate, and in the case that the adhesion pad 4 is detached from between the pins 16 in the collection step as in the first example, through-holes 4a are kept formed at parts where the above-described pins 16 penetrate in the collected adhesion pad 4.

Then, in the present example, when the cell aggregates 2 are cultured and fused to some extent and the adhesion pad 4 in the middle of the culture is obtained, the incubator 6 is connected to the isolator 5 and the culture container 3 is mounted on the above-described culture container supporting section 9 inside the isolator 5.

Then, in the culture container supporting section 9, the operator or the robot or the like raises the support plate 17 positioned at the above-described lowered positions to the raised position, and detaches the adhesion pad 4 in the middle of the culture from between the pins 16.

Subsequently, as is shown in FIG. 7(A), the adhesion pad 4 in the state of being mounted on the support plate 17 is moved such that, for example, a center position of the cell aggregate 2 constituting the adhesion pad 4 is positioned above the distal end of the pin 16.

Thereafter, when the above-described support plate 17 is moved again from the raised position to the lowered position as shown in FIG. 7(B), only the support plate 17 is lowered along the above-described pins 16, and the above-described adhesion pad 4 in the middle of the culture is supported from below by the above-described pins 16.

When this state is attained, the culture container 3 is transferred again from the isolator 5 to the incubator 6 and the culture step is restarted. Thus, the above-described through-holes 4a are filled.

In addition, since the adhesion pad 4 is supported from below by the above-described pins 16, the gap g is formed at the lower part of the adhesion pad 4, and the culture liquid is continuously supplied to the lower part of the adhesion pad 4.

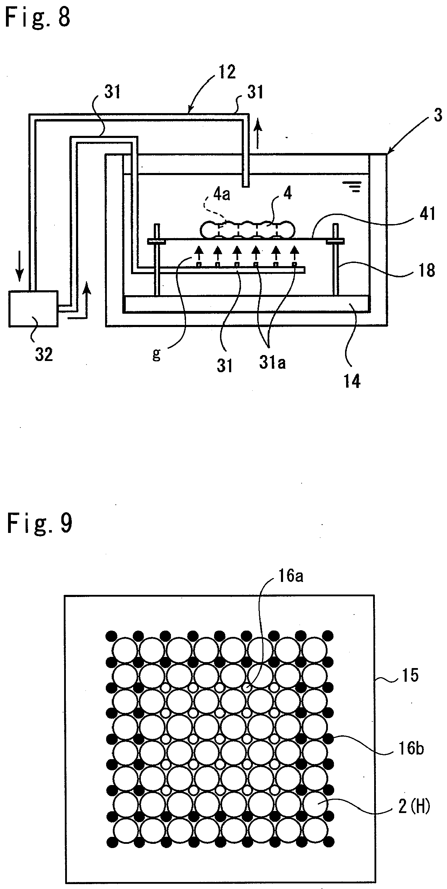

FIG. 8 is a diagram for describing a method for producing a cell mass structure as a third example, and is specifically the work that is executed in the middle of the above-described culture step in the above-described first example similarly to the above-described second example, and the same elements are used for the culture container 3 and the like.

The step relating to the present example is also performed after the cell aggregates 2 are fused to some extent and the adhesion pad 4 in the middle of the culture is formed, similarly to the above-described second example.

The culture container 3 accommodating the adhesion pad 4 in the middle of the culture is transferred from the incubator 6 to the isolator 5, and in the isolator 5, a new culture container 3 is prepared. The culture container 3 does not include the accommodating means formed of the above-described pedestal 15 and the pins 16, and instead of the above-described support plate 17, a mesh-like support plate 41 is provided on a position separated upwards from the bottom face plate 14 by the guide members 18.

The operator takes out the adhesion pad 4 in the middle of the culture held between the above-described pins 16 from the inside of the culture container 3 transferred from the incubator 6 using the above-described support plate 17, and transfers it onto the support plate 41 of the above-described new culture container 3.

Thereafter, the culture container 3 accommodating the adhesion pad 4 in the middle of the culture is transferred to the incubator 6, and the culture step is restarted.

In addition, the culture liquid supply means 12 shown in FIG. 8 has a plurality of injection holes 31a provided on the pipe 31 positioned in the gap g below the above-described adhesion pad 4, and discharges the culture liquid from the plurality of injection holes 31a.

Thus, the flow of the culture liquid can be generated in a wide range to the bottom face of the adhesion pad 4, and the culture liquid can be brought into contact with the cells on the bottom face side constituting the adhesion pad 4 better.

Incidentally, the structure of the above-described culture liquid supply means 12 in the present example can be applied even to the above-described first and second examples.

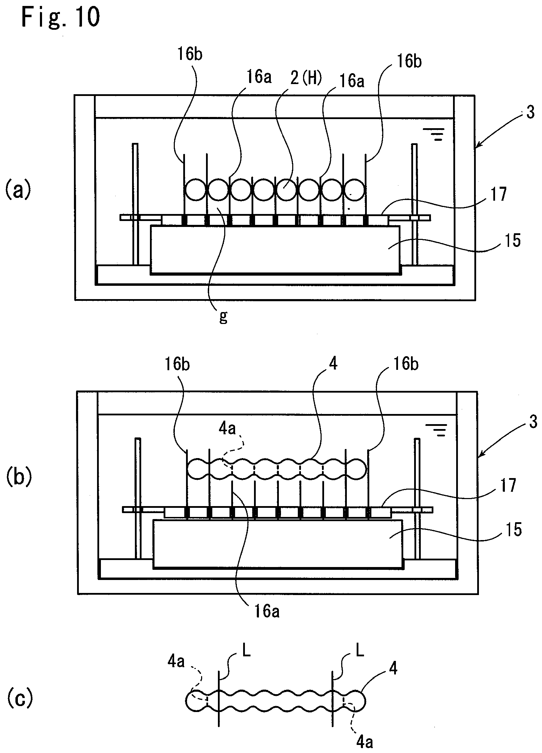

FIG. 9 to FIG. 12 are diagrams for describing a method for producing a cell mass structure as a fourth example, and among them, FIG. 10 shows the work that is executed in the middle of the above-described culture step in the above-described first example and the work that is executed upon the above-described collection work, similarly to the above-described second and third examples.

In the culture container 3 used in the present example, the pins 16 fixed to the above-described pedestal 15 are structured by a first pin group (16a) that holds the center portion of the adhesion pad 4, and a second pin group (16b) that holds an outer peripheral edge portion of the adhesion pad 4 and illustrated in black.

Specifically, in FIG. 9, the pins 16b structuring the above-described second pin group are arranged in a frame shape of being arranged on the outermost periphery and one line on the inner side. Therefore, when the cell aggregates 2 are accommodated in the culture container 3 in the supply step, the cell aggregates 2 arranged on the outermost periphery are surrounded and held by the pins 16b of the above-described second pin group.

In addition, as is shown in FIG. 10, the pins 16a structuring the above-described first pin group are shorter than the pins 16b structuring the above-described second pin group, and a height of the accommodating portions H that hold the above-described cell aggregates 2 is set at the height near the distal end portion of the pins 16a of the above-described first pin group.

By such a structure, similarly to the above-described individual examples, the gap g can be formed with the pedestal 15 below the cell aggregates 2 held by the above-described accommodating portions H.

The method for producing a cell mass structure using the above-described culture container 3 will be described. As is shown in FIG. 10(A), in the supply step, similarly to the above-described first example, the cell aggregates 2 are held by the accommodating portions H of the pins 16a and 16b of the above-described first and second pin groups.

Thereafter, the culture step is performed similarly to the above-described second and third examples, and when the adhesion pad 4 in the middle of the culture in which the cell aggregates 2 are fused to some extent is formed in the middle, the culture container 3 accommodating the adhesion pad 4 in the middle of the culture is transferred from the incubator 6 to the isolator 5.

Then, as is shown in FIG. 10(B), the support plate 17 positioned at the lowered position is raised, and the above-described adhesion pad 4 is moved to the height near the distal end portion of the pins 16b of the second pin group.

Thus, the pins 16a of the above-described first pin group are detached from the above-described adhesion pad 4 in the middle of the culture, and the adhesion pad 4 is held by the pins 16b of the above-described second pin group.

Thereafter, similarly to the above-described second and third examples, the adhesion pad 4 in the middle of the culture is again transferred from the isolator 5 to the incubator 6, and the culture step is restarted.

At the time, since the through-holes 4a by the pins 16a of the first pin group that have penetrated until then are formed at the center portion of the adhesion pad 4, by restarting the culture step, the above-described through-holes 4a are filled with the cells.

Here, when the above-described through-holes 4a are filled with the cells, there is a risk that the cells are attracted to each other and the adhesion pad 4 is contracted toward the center portion and curved.

However, in the present example, the outer peripheral edge portion of the adhesion pad 4 is held by the pins 16b of the above-described second pin group, contraction of the center portion of the adhesion pad 4 is prevented, and the adhesion pad 4 of the uniform thickness without being curved can be obtained.

When the above-described culture step ends, and the through-holes 4a formed at the center portion of the above-described adhesion pad 4 are filled with the cells, the above-described culture container 3 is transferred from the incubator 6 to the isolator 5, and the above-described collection step is performed thereafter.

In the collection step, the above-described support plate 17 is raised again from the lowered position to the raised position, and the adhesion pad 4 is detached from the pins 16b of the above-described second pin group.

Since the through-holes 4a by the pins 16b of the above-described second pin group are formed at the outer peripheral edge portion of the adhesion pad 4 collected in this way, in the collection step of the present example, as is shown in FIG. 10(C), an outer peripheral edge portion L of the adhesion pad 4 is cut off and removed, and only the center portion is collected.

Specifically, as cutting means that cuts off the outer peripheral edge portion of the adhesion pad 4, for example, a surgical knife used by the operator wearing the gloves inside the isolator or a laser cutter provided outside the isolator can be used.

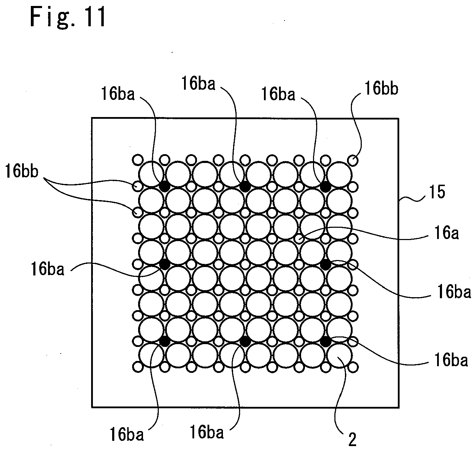

Incidentally, among the pins 16 described using FIG. 9 described above, the pins 16b structuring the above-described second pin group can also be structured as shown in FIG. 11.

While all the pins 16b of the second pin group are of the same length in FIG. 9, in FIG. 11, only pins 16ba indicated in black in the figure among the pins 16b of the second pin group are made long, and the other pins 16bb are of the same length as that of the pins 16a of the above-described first pin group.

Describing specifically, the above-described long pins 16ba are provided respectively on four vertex portions and center portions of individual sides, among the pins 16b arrayed in an approximate square on one line inner side of the pins 16b provided on the outermost periphery.

In the case that the second pin group is structured in this way, when the pins 16a of the first pin group are detached from the adhesion pad 4 in the middle of the above-described culture step, the short pins 16bb of the above-described second pin group are also detached from the adhesion pad 4.

Even in that case, since the above-described long pins 16ba hold the outer peripheral edge portion of the adhesion pad 4, the contraction when the through-holes 4a are filled with the cells at the center portion of the above-described adhesion pad 4 can be prevented. Incidentally, the arrangement of the long pins 16ba in the above-described second pin group can be appropriately set according to the shape of the adhesion pad 4.

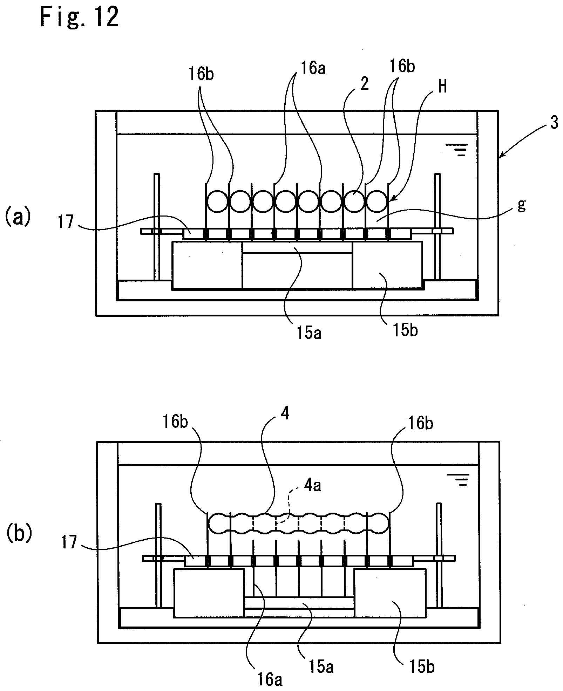

Further, in the above-described fourth example, the culture container 3 having the structure shown in FIGS. 12 can be used. In the structure, the pins 16a structuring the above-described first pin group and the pins 16b structuring the second pin group are formed in the same length.

Then, the pins 16a of the above-described first pin group and the pins 16b of the second pin group are respectively provided on a first pedestal 15a and a second pedestal 15b, and the second pedestal 15b is provided so as to surround the first pedestal 15a.

Then, the first pedestal 15a provided with the pins 16a of the above-described first pin group is provided so as to be moved up and down, and a not-shown grasping member that can be operated by the operator or the robot is fixed to the first pedestal 15a.

As is shown in FIG. 12(A), in the above-described supply step, the above-described first pedestal 15a is positioned at the raised position, and the pins 16a structuring the first pin group are held at the same height at that of the pins 16b structuring the second pin group.

Therefore, by setting the accommodating portions H near the distal end portions of the individual pins 16, the gap g is formed between the held cell aggregates 2 and the first and second pedestals 15a and 15b.

Then, when the adhesion pad 4 in the middle of the culture is obtained in the middle of the culture step, the above-described first pedestal 15a is lowered as shown in the FIG. 12(B), and thereby the pins 16a of the first pin group are detached from the adhesion pad 4, and the adhesion pad 4 in the middle of the culture is held by the pins 16b of the second pin group.

Thereafter, when the adhesion pad 4 is collected in the collection step, the above-described support plate 17 is raised, and thereby the adhesion pad 4 can be detached from the pins 16b of the second pin group.

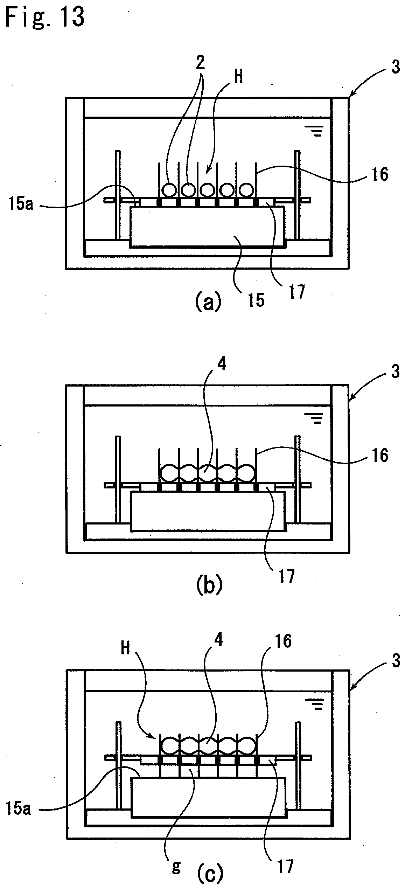

FIG. 13 is diagrams for describing a method for producing a cell mass structure as a fifth example, that is executed instead of the above-described supply step in the above-described first example and is executed in addition to the culture step further.

The present example copes with the case in which the diameter of the cell aggregate 2 is smaller than the interval of the pins 16 at the individual diagonal positions in the accommodating portion H and the cell aggregate 2 cannot be held between the pins 16 and the pins 16.

Specifically, the case of using the cell aggregates 2 as small as possible in order to thin the adhesion pad 4 to be produced and the case that the size of the produced cell aggregates 2 varies and the cell aggregates 2 that fall down from the accommodating portions H are included are assumed.

Then, in the supply step of the present example, as shown in FIG. 13(A), the above-described suction nozzles 10 mount the cell aggregates 2 on the upper face of the support plate 17 positioned on the installation surface 15a of the above-described pedestal 15.

Then, while keeping the state that the above-described cell aggregates 2 are mounted on the support plate 17, the culture container 3 is transferred to the incubator 6 to start the above-described culture step, and the cell aggregates 2 are cultured until becoming the size that can be held in the accommodating portions H between the pins 16 as shown in FIG. 13(B).

In this way, when the cell aggregates 2 with the sufficient size that can be held in the accommodating portions H are obtained, the culture container 3 is transferred to the isolator 5, and the support plate 17 inside the culture container 3 is raised as shown in FIG. 13(C).

Then, the above-described cell aggregates 2 in the middle of the culture are raised along the pins 16 together with the support plate 17, and the cell aggregates 2 in the middle of the culture are held in the accommodating portions H set at the vertical middle position of the above-described pins 16. Thereafter, the above-described support plate 17 is lowered and retracted below.

Thus, similarly to the above-described first example, since the cell aggregates 2 can be held in the accommodating portions H set separated from the above-described installation surface 15a where the plurality of pins 16 are erected, in the case that the culture step is restarted thereafter, by distributing the culture liquid in the gap g formed with the installation surface 15a, the culture liquid can be sufficiently brought into contact with the lower portion of the adhesion pad 4 in the middle of the culture.

Here, by making the surface of the above-described support plate 17 be non-adhesive to cells, the adhesion pad 4 in the middle of the culture can be easily detached from the support plate 17. Here, non-adhesion to cells can be obtained by making properties of the surface of the support plate 17 be water-repellent or super-hydrophilic, and can be realized by adopting a well-known material having the properties or executing surface treatment or coating treatment. In addition, it is also possible to form a DLC (diamond-like carbon) film.

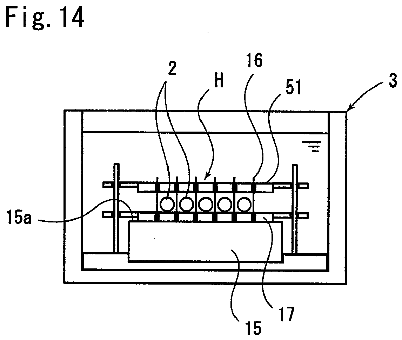

For the method for producing a cell mass structure relating to the above-described fifth example, between the supply step in FIG. 13(A) and the culture step in FIG. 13(B), a step may be added that arranges a contact plate 51 as an upper contact member at the upper portion of the cell aggregates 2 supported by the support plate 17 as the above-described lower support member as shown in FIG. 14.

The above-described contact plate 51 has a structure similar to that of the support plate 17, and is provided so as to be raised and lowered in the Z direction along the above-described pins 16 and the guide members 18, and the back face side to be in contact with the cell aggregates 2 is non-adhesive to cells.

A using method will be described. When the above-described suction nozzles 10 mount the cell aggregates 2 on the upper face of the support plate 17 in the supply step in FIG. 13(A), the above-described contact plate 51 is mounted on the upper portion of the mounted cell aggregates 2 by the operator or the robot, and the cell aggregates 2 are supported in the state of being held by the support plate 17 and the contact plate 51.

By mounting the contact plate 51 in this way, the float of the cell aggregates 2 not held immovably by the four pins 16 can be prevented, and the shape of the adhesion pad 4 in which the cell aggregates 2 are fused can be adjusted.

In this state, the culture container 3 is transferred to the incubator 6 to start the above-described culture step, and the cell aggregates 2 are cultured until they become the size to be supported between the pins 16 and the adhesion pad 4 in the middle of the culture in which the adjacent cell aggregates 2 are fused is obtained, as shown in FIG. 13(B).

When the cell aggregates 2 in the sufficient size are obtained in this way, the culture container 3 is transferred to the isolator 5, and the support plate 17 inside the culture container 3 is raised as shown in FIG. 13(C). At the time, the above-described contact plate 51 may be removed or kept mounted as it is.

Further, in the case that the diameter of the cell aggregate 2 is smaller than the interval of the pins 16 at the individual diagonal positions of the accommodating portion H and the cell aggregate 2 cannot be held at the vertical middle position of the pins 16 as in the above-described fifth example, in the above-described fourth example, the support plate 17 may be positioned corresponding to the height of the accommodating portions H set at the required height beforehand.

That is, the cell aggregates 2 are mounted on the upper face of the support plate 17 positioned in accordance with the height of the accommodating portions H, and the individual cell aggregates 2 are respectively cultured to the size that can be held in the accommodating portions H in this state. Thus, when the individual cell aggregates 2 are turned to the state of being held in the accommodating portions H, the support plate 17 is retracted below, and the above-described gap g is formed below the adhesion pad 4 in the middle of the culture.

Even in that case, by mounting the above-described contact plate 51 on the upper portion of the cell aggregates 2 mounted on the above-described support plate 17 and holding and supporting the cell aggregates 2 by the support plate 17 and the contact plate 51, the float of the cell aggregates 2 is prevented, and the culture can be performed to adjust the shape of the adhesion pad 4.

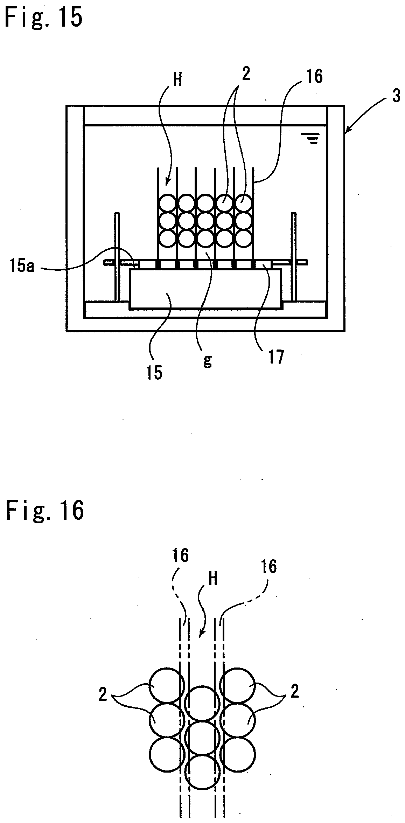

FIGS. 15 and 16 are diagrams for describing a method for producing a cell mass structure relating to a sixth example, and describe a method for producing a three-dimensional cell mass structure in which the cell aggregates 2 are arrayed in the Z direction in the present example in contrast with the planar adhesion pad 4 as the cell mass structure produced in the above-described first to fifth examples.

However, even in the case of producing the three-dimensional cell mass structure, it is possible to use the apparatus including the structure similar to that of the apparatus 1 for producing the cell mass structure used in the above-described first example, and since the three-dimensional cell mass structure can be produced using the approximately similar steps, detailed descriptions will be omitted.

In the culture container 3 used in the present example shown in FIG. 15, the length of the above-described pins 16 is set in accordance with the height of the three-dimensional cell mass structure that is desired, and it is possible to hold the plurality of cell aggregates 2 in the Z direction in the accommodating portion H formed by the four pins 16.

Then, in the above-described supply step, the above-described suction nozzles 10 supply the above-described cell aggregates 2 to the accommodating portions H formed by the above-described pins 16, based on the three-dimensional arrangement of the cell aggregates 2 set beforehand.

Specifically, firstly the culture container supporting section 9 successively moves the culture container 3 in the Y direction, the cell aggregates 2 to be arranged in the bottom stage are supplied, and the cell aggregates 2 are successively supplied to the upper portion of the cell aggregates 2 held in the accommodating portions H thereafter.

At this time also, similarly to the above-described individual examples, the gap g is formed below the cell aggregates 2 positioned in the bottom stage, and thereby the culture liquid is supplied to the lower face of the three-dimensional cell mass structure.

In the present example, since the accommodating means including the above-described pins 16 is positioned at the approximate center of the above-described culture container 3, the gap g is formed also between a side face portion of the three-dimensional cell mass structure supported by the above-described pins 16 and a side face portion of the culture container 3, and the culture liquid is supplied also to the side face portion of the cell mass structure.

In addition, in the case of producing the above-described three-dimensional cell mass structure, it is conceivable to turn the heights of the cell aggregates 2 held in the adjacent accommodating portions H to the heights shifted by a half of the cell aggregate 2, for example.

As already described, since the cells structuring the cell aggregates 2 supported by the above-described pins 16 increase along the periphery of the above-described pins 16, by making the heights of the cell aggregates 2 in the adjacent accommodating portions H be different as in FIG. 16, the cell aggregates 2 can be efficiently fused.

Incidentally, also in the case of producing such a three-dimensional cell mass structure, it is possible to use the work performed in the second to fifth examples.

Incidentally, in the above-described individual examples, as the arrangement of the pins 16 that support the above-described cell aggregates 2, as long as the cell aggregate 2 can be supported by the plurality of pins 16, the pins 16 may be arranged so as to surround each cell aggregate 2 by six pins 16, for example.

In that case, the above-described accommodating portions H are arranged in zigzag, and it is also possible to produce the three-dimensional cell mass structure as in the sixth example using this arrangement.

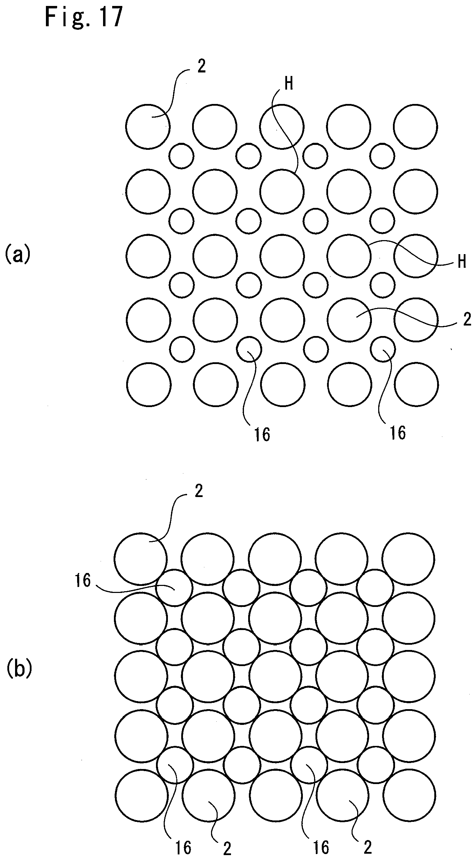

Further, in the above-described individual examples, in accordance with the thickness of the pins 16 provided in the culture container 3, the size of the accommodating portions H formed by the pins 16, or the diameter of the cell aggregates 2 to be used, various aspects are conceivable as shown in FIG. 17.

FIG. 17(A) illustrates the case in which the diameter of the cell aggregate 2 is smaller than the interval of the two pins 16 at the diagonal positions and the cell aggregate 2 cannot be held by the four pins 16.

Even in this case, the cell aggregates are just mounted on the above-described support plate in the above-described supply step, and though the cell aggregates 2 in the adjacent accommodating portions H are not in contact with each other at the time, as shown in the above-described fifth example, since the cell aggregates 2 are cultured in the culture step so that the adjacent cell aggregates 2 are fused, the cell mass structure can be obtained.

In addition, FIG. 17(B) illustrates the case in which the diameter of the above-described pins 16 is larger than the diameter of the pins 16 shown in the above-described individual examples and the adjacent cell aggregates 2 held in the above-described accommodating portions H do not come into contact with each other even in the case that the diameter of the cell aggregate 2 is larger than the interval of the two pins 16 at the diagonal positions.

Even in such a case, since the cell aggregates 2 are cultured in the above-described culture step and the adjacent cell aggregates 2 are fused, the cell mass structure can be obtained.

### Reference Signs List

1 Apparatus for producing cell mass structure
2 Cell aggregate
3 Culture container
4 Adhesion pad (cell mass structure)
10 Suction nozzle
12 Culture liquid supply means
16 Pin
17 Support plate (lower support member) H Accommodating portion
g Gap

## Claims

1. A method for producing a cell mass structure, which produces a cell mass structure in which a plurality of cell aggregates arranged inside a culture container accommodating a culture liquid are fused to each other by being cultured, **characterized in that**
a plurality of pins are erected inside the culture container, and the cell aggregates are accommodated by the plurality of pins with parts between the plurality of pins and pins as accommodating portions, and
the cell aggregates are cultured in a state of being accommodated in a plurality of adjacent accommodating portions.

2. The method for producing the cell mass structure according to claim 1, **characterized in that**
by performing arrangement such that an interval of two pins at diagonal positions becomes shorter than a diameter of the cell aggregate, the cell aggregate can be held by the accommodating portion, and the accommodating portion is set at a position away from an installation surface where the plurality of pins are erected,
thereby forming a gap through which the culture liquid can be distributed between the cell aggregate held by the accommodating portion and the installation surface.

3. The method for producing the cell mass structure according to claim 1, **characterized in that**
the culture container comprises a lower support member that supports the cell aggregate from below between the plurality of pins and pins,
wherein the cell aggregate is cultured in a state of being supported by the lower support member, and when the cell aggregate can be held by the accommodating portion, the cell aggregate is held by the accommodating portion and the lower support member is retracted.

4. The method for producing the cell aggregates according to any one of claim 1 to claim 3, **characterized in that**
the pins are structured by a first pin group that holds a center portion of the cell mass structure, and a second pin group that holds an outer peripheral edge portion of the cell mass structure, and
when the cell aggregate is cultured and the cell mass structure in the middle of being cultured is formed, the pins belonging to the first pin group are detached from the cell mass structure, and the outer peripheral edge portion of the cell mass structure is held by the pins belonging to the second pin group.

5. The method for producing the cell mass structure according to claim 4, **characterized in that**
when the cell mass structure is obtained, the outer peripheral edge portion of the cell mass structure held by the second pin group in the cell mass structure is removed by cutting means.

6. The method for producing the cell aggregates according to any one of claim 1 to claim 5, **characterized in that**
the cell aggregates are planarly arranged inside the culture container, and an adhesion pad in which the plurality of the cell aggregates are fused is produced.

7. An apparatus for producing a cell mass structure, which produces a cell mass structure in which a plurality of cell aggregates arranged inside a culture container accommodating a culture liquid are fused to each other by being cultured, **characterized by** comprising:
accommodating means with a plurality of pins erected and provided inside the culture container; and
supply means that supplies the cell aggregates between the plurality of pins and pins in the accommodating means further,
wherein, with parts between the plurality of pins and pins as the accommodating portions of the cell aggregates, the supply means accommodates the aggregates in the plurality of adjacent accommodating portions.

8. The apparatus for producing the cell mass structure according to claim 7, **characterized in that**
by performing arrangement such that an interval of the two pins at diagonal positions becomes shorter than a diameter of the cell aggregate, the cell aggregate can be held by the accommodating portion, and the accommodating portion is set away from an installation surface where the plurality of pins are erected, and
the accommodating portion is made to hold the cell aggregate by the supply means.

9. The apparatus for producing the cell mass structure according to any one of claim 7 and claim 8, **characterized by** comprising
a lower support member that supports the cell aggregate from below between the plurality of pins and pins, and is movable along the pins.

10. The apparatus for producing the cell mass structure according to claim 9, **characterized by** comprising
an upper contact member that comes into contact with an upper portion of the cell aggregate supported by the lower support member,
wherein the cell aggregate is supported in a state of being held by the lower support member and the upper contact member.

11. The apparatus for producing the cell mass structure according to any one of claim 7 to claim 10, **characterized in that**
the pins are structured by a first pin group that holds a center portion of the cell mass structure, and a second pin group that holds an outer peripheral edge portion of the cell mass structure,
the pins structuring the first pin group are formed shorter than the pins structuring the second pin group, the accommodating portion that holds the cell aggregate is set near a distal end portion of the first pin group,
the pins structuring the first pin group and the second pin group are provided integrally and relatively movably to the cell mass structure further, and
in the state that the cell aggregate is cultured and the cell mass structure in the middle of being cultured is formed, by movement of the pins structuring the first pin group and the second pin group, the pins belonging to the first pin group are detached from the cell mass structure, and the pins structuring the second pin group hold the cell mass structure.

12. The apparatus for producing the cell mass structure according to any one of claim 7 to claim 10, **characterized in that**
the pins are structured by a first pin group that holds a center portion of the cell mass structure, and a second pin group that holds an outer peripheral edge portion of the cell mass structure,
the pins structuring the first pin group and the second pin group are provided relatively movably, and
in the state that the cell aggregate is cultured and the cell mass structure in the middle of being cultured is formed, the pins structuring the first pin group are moved and detached from the cell mass structure, and the pins structuring the second pin group hold the cell mass structure.
